(19) Europäisches Patentamt
European Patent Office
Office européen des brevets

(11) **EP 3 093 678 A1**

(12) **EUROPÄISCHE PATENTANMELDUNG**

(43) Veröffentlichungstag:
**16.11.2016 Patentblatt 2016/46**

(51) Int Cl.:
*G01R 33/28* (2006.01)          *G01R 33/563* (2006.01)
*A61B 5/00* (2006.01)

(21) Anmeldenummer: **15167568.3**

(22) Anmeldetag: **13.05.2015**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA**

(71) Anmelder: **Bayer Pharma Aktiengesellschaft 13353 Berlin (DE)**

(72) Erfinder: **Rohrer, Martin 12203 Berlin (DE)**

(74) Vertreter: **BIP Patents c/o Bayer Intellectual Property GmbH Alfred-Nobel-Straße 10 40789 Monheim am Rhein (DE)**

Bemerkungen:
Geänderte Patentansprüche gemäss Regel 137(2) EPÜ.

(54) **VERFAHREN ZUR OPTIMIERUNG DER VORBESTIMMUNG DES ZEITLICHEN VERLAUFES EINER KONTRASTMITTELKONZENTRATION BEI DER DIAGNOSTISCHEN BILDGEBUNG MIT EINEM MAGNETRESONANZSYSTEM**

(57)   Die Erfindung betrifft ein Verfahren zur Optimierung der Vorbestimmung des zeitlichen Verlaufes einer Kontrastmittelkonzentration an einer Gefäßposition in einer Untersuchungsumgebung (ROI) bei einer kontrastmittelgestützten MR-Aufnahme eines Patienten (P) ausschließlich in der ersten Anflutungsphase des Kontrastmittels, wobei eine Verbreiterung ($\Delta W = W2 - W1$) eines Kontrastmittelbolusverlaufes (B(t)) mit einer ersten Breite (W1) an einer vorgegebenen ersten Gefäßposition (P1) eines Patienten (P) zu einem Kontrastmittelkonzentrationsverlauf (K(t)) mit einer zweiten Breite (W2) an einer vorgegebennen zweiten Gefäßposition (P2), die in der Untersuchungsumgebung (ROI) angeordnet ist, durch Bestimmung mindestens eines, zumindest von einer Blutflusseigenschaft ($f_B$) an einer dritten Gefäßposition (P3) abhängigen und mit der Verbreiterung korrelierten, Flussparameters ($P_G$) ermittelt wird.

Weiterhin betrifft die Erfindung auch ein Magnetresonanzsystem zur Erzeugung einer kontrastmittelunterstützten MR-Aufnahme oder einer MR-Gefäßdarstellung mit einem, von einem Computer gesteuerten Kontrastmittelinjektor, wobei der Computer mit einem Speicher für Programme, die im Betrieb ausgeführt werden, ausgestattet ist und Programme gespeichert sind, welche das erfindungsgemäße Verfahren ausführen.

FIG 2

arterieller Kreislauf / *arterial circulation*
venöser Kreislauf / *venous circulation*
Lunge / *lung*
Herz / *heart*
innere Organe / *inner organs*
periphere Gefäße / *peripheral vessels*
P1, P2, P3, P4, ROI

**Beschreibung**

Die Erfindung betrifft ein Verfahren zur Optimierung der Vorbestimmung des zeitlichen Verlaufes einer Kontrastmittelkonzentration an einer Gefäßposition in einer Untersuchungsumgebung bei einer kontrastmittelgestützten MR-Aufnahme eines Patienten ausschließlich in der ersten Anflutungsphase des Kontrastmittels.

In der diagnostischen Bildgebung sind Verfahren der Magnetresonanztomographie (MRT) allgemein bekannt. Insbesondere zur Darstellung von Blutgefäßen in der Magnetresonanz-Angiographie (MRA) wird zur Hervorhebung der arteriellen Gefäße gegenüber dem restlichen Gewebe ein Kontrastmittel eingesetzt. Solche Verfahren werden als kontrastmittelverstärkte MR-Angiographie (CE-MRA) bezeichnet. Werden Darstellungen von peripher gelegenen Blutgefäßen, wie beispielsweise Beinarterien, aufgenommen, spricht man von kontrastmittelverstärkter peripherer MR-Angiographie (CE-pMRA).

Die CE-MRA stellt im Vergleich zu anderen, nicht durch Kontrastmittel verstärkten Bildgebungsmethoden, in vielen klinischen Fällen die bevorzugte Methode einer strahlungsfreien, nicht-invasiven Gefäßdiagnostik dar. Sie wurde vor ca. 20 Jahren erstmals durchgeführt, seitdem kontinuierlich technisch verbessert und stellt heute in den meisten Ländern ein fortgeschrittenes diagnostisches Standardverfahren dar.

Die klinischen Fragestellungen erfordern in den meisten Fällen eine gegenüber dem umliegenden Gewebe möglichst kontrastreiche Visualisierung des *arteriellen* Gefäßsystems, wobei die gleichzeitige Visualisierung des *venösen* Gefäßsystems in der Regel als störende Überlagerung gesehen wird. Daher betrifft das hier beschriebene erfindungsgemäße Verfahren auch ausschließlich kontrastmittelgestützte MR-Untersuchungen, bei denen nur die erste Anflutungsphase des Kontrastmittels zur Bilddarstellung genutzt wird, also sogenannte "First Pass"-MR-Untersuchungen und es ist wesentlich , venöse Überlagerungen durch technische Maßnahmen und Optimierungen zu vermeiden oder so gering wie möglich zu halten.

Hierfür ist grundsätzlich eine möglichst sekundengenaue Synchronisation des im Gefäßsystem der Zielregion anflutenden Kontrastmittels mit dem Beginn der Datenakquisition wichtig, sowohl um die verfügbare Kontrastverstärkung durch das Kontrastmittel voll ausnutzen zu können als auch, um der anschließenden Rückflutung des Kontrastmittels im venösen Gefäßsystem zuvor zu kommen. Es wird also, im Gegensatz zur Anwendung von Kontrastmitteln in der Computertomographie (CT) beziehungsweise CT-Angiographie, in der die Blutgefäße einer zu untersuchenden Region im typischen Bereich von ca. 25 - 50 Sekunden mit Kontrastmittel geflutet werden, das Kontrastmittel über eine wesentlich kürzere Zeitspanne im typischen Bereich von ca. 3 bis 15 Sekunden appliziert.

Zu erwähnen ist außerdem, dass bei der kontrastmittelgestützten MR-Untersuchung im Gegensatz zur kontrastmittelgestützten fluoroskopischen Röntgen- oder CT-Untersuchung kleinere Volumina betrachtet werden, eine grundsätzlich unterschiedliche Akquisitionstechnik mit Besonderheiten der K-Raum-Akquisition und Fourier-Transformation, andere Messzeiten und Anforderungen an Kontrastmittelkonzentration während der Messung und eine nichtlineare Signalintensität als Funktion der lokalen Kontrastmittelkonzentration im Untersuchungsbereich vorliegt. Eine direkte Übertragung der Verhältnisse in der CT bei der Kontrastmittelapplikation auf diejenigen in der MRT ist daher in der Regel nicht möglich.

Für die Synchronisation eines applizierten Kontrastmittelbolus in der CE-MRA (*bolus timing*) sind mehrere verschiedene Methoden bekannt.

a. Verwendung eines Testbolus zur prospektiven Bestimmung der voraussichtlichen Dauer, die der injizierte Kontrastmittelbolus von der i.v.-Injektion bis zur Zielregion im Körper benötigt (Boluslaufzeit / *bolus travel time* = BTT, Bolusankunftszeit / *bolus arrival time* = BAT, Bereich von Interesse / *region of interest* = ROI).

Hierbei wird vor der eigentlichen, CE-MRA, die BTT mittels eines Testbolus gemessen. Der Testbolus hat dabei typischerweise ca. 10%-15% des eigentlichen Kontrastmittelvolumens. Die Untersuchung beinhaltet meistens eine vorgelagerte Prozedur, bestehend aus der zeitaufgelösten 2D-Messung eines zuvor sorgfältig gesetzten Akquisitionsfensters in der Zielregion. Dabei misst man die vom i.v. injizierten Testbolus beeinflusste Signalintensität *(signal intensity, SI)* quantitativ innerhalb einer großen Arterie meist unterhalb der Aortenbifurkation (z.B. A. iliaca, A. poplitea). Die SI wird als Funktion der Zeit mit einer zeitlichen Auflösung von typischerweise 1 Sekunde ab dem Zeitpunkt der Testbolusinjektion gemessen. Grundsätzlich gilt der Beginn einer Injektion als der Startzeitpunkt. Die Testbolusinjektion erfolgt in der Regel mit einer auch für die eigentliche Kontrastmittelinjektion vorgesehenen Injektionsrate (z.B. 1 mL/s), gefolgt von einer Kochsalzinjektion. Aus der so gemessenen Anflutungskurve des Testbolus in der gewählten Körperregion kann die BTT und auch die Verbreiterung eines Bolus im Zielgebiet entnommen werden. Ein solches Verfahren ist weitgehend robust und allgemein etabliert. Bei richtiger Anwendung durch erfahrenes Personal führt es in den meisten Fällen zu guten Ergebnissen. Allerdings ist das Verfahren zeitaufwendig, da einige Minuten für die Testbolusmessung und deren Auswertung verloren gehen. Auch erfahrene Anwender berichten dahingehend von Problemen im mindestens einstelligen Prozentbereich der Anwendungen, dass eine korrekte Testbolusdetektion sehr schwierig oder nicht möglich ist. Die Ursachen dafür sind nicht eindeutig bekannt. Möglich sind zu starke Bolusdispersion und zu starke Verdünnungseffekte aufgrund spezifischer kardio-vaskularer Verhältnisse. Weitere Nachteile dieses Verfahrens sind der zusätzliche Kontrastmittelverbrauch, beziehungsweise eine zu geringe Dosierung bei der eigentlichen KontrastmittelUntersuchung zur Kompensation des zuvor applizierten Testbolus. Außerdem kann der vor der Untersuchung applizierte Testbolus zu unerwünschten Hintergrundeffekten führen, da sich bei der eigentlichen Untersuchung bereits Kontrastmittel aus dem Testbolus im Interstitium befindet.

b. "Fluoroskopische" Methoden zur direkten Bestimmung der Kontrastmittelanflutung in der ROI, ohne vorherige Messung der BTT.

Ähnlich wie bei der dynamischen Messung des Testbolus nach a. kann eine geeignet gewählte zweidimensionale Bilddarstellung auch dazu verwendet werden, die Anflutung des eigentlichen Kontrastmittelbolus in Echtzeit unmittelbar vor Erreichen der Zielregion zu beobachten. Dabei ist es wichtig, dass eine geeignete Region strömungsmäßig knapp *oberhalb* der eigentlichen ROI mit einer Zeitauflösung im Bereich von mindestens einer Sekunde betrachtet wird, so dass die Anflutung zuverlässig und rechtzeitig erkannt wird. Sobald eine Detektion der Bolusanflutung erfolgt, muss innerhalb sehr kurzer Zeit das Messprogramm von zeitaufgelöster zweidimensionaler Akquisition auf die gewünschte, in der Regel dreidimensionale, Akquisition mit hoher räumlicher Auflösung umgeschaltet werden. Hierfür sind verschiedene, teils herstellerspezifische und automatisierte Verfahren der Datenakquisition bekannt.

Günstig ist hierbei, dass man ohne Zeit- und Kontrastmittel-Verlust auskommt, da eine Testbolusmessung nicht notwendig ist. Nachteilig ist allerdings, dass grundsätzlich eine verbleibende Ungenauigkeit der tatsächlichen BAT in der Zielregion verbleibt, weil die Kontrastmittelanflutung immer kurz oberhalb der Zielregion beobachtet werden muss, um noch die technisch notwendige Zeitspanne einiger Sekunden für das Umschalten von der zeitaufgelösten 2D-Bolusdetektion zur 3D-Akquisition der ROI mit hoher räumlicher Auflösung sicherzustellen. Diese verbleibende, unbekannte Zeitspanne muss durch Erfahrungswerte und Faustregeln ersetzt werden, was aufgrund der teilweise sehr starken, individuellen Physiologie und kardiovaskulärer Pathologien zumindest in Einzelfällen zu Verschlechterungen der Ergebnisse führt.

c. "4D" Datenakquisition ohne vorherige Bestimmung der Bolusankunftszeit und ohne spezifische Triggerung der Messung durch den Kontrastmittelbolus.

Bei diesen Verfahren wird grundsätzlich ein Kompromiss zwischen räumlicher und zeitlicher Auflösung der erzielten Bilddaten in Kauf genommen, indem die Kontrastmittelanflutung mit einer dynamisch wiederholten Akquisition der dreidimensionalen Zielregion, beispielsweise jeweils innerhalb von 3 bis 5 Sekunden, erreicht wird. Die räumliche Auflösung ist bei diesen kurzen Messzeiten allerdings begrenzt und beträgt typischerweise mindestens 2mm - 3mm. Bei einer 3D-Akquisition mit hoher räumlicher Auflösung, z.B. 1mm in allen Raumrichtungen oder besser, beträgt die Messzeit meistens ca. 15 Sekunden. Durch die dynamisch wiederholten Messungen wird der Kontrastmittelbolus auf jeden Fall aufgenommen, meist sogar mit mehreren 3D-Datensätzen. Eine Bolus-Synchronisation ist somit überflüssig. Vorteilhaft ist die bei diesen Verfahren zusätzlich gewonnene dynamische Information der Kontrastmittelanflutung, nachteilig jedoch die schlechtere räumliche Auflösung.

Trotz erheblichen technischen Fortschritts während der letzten Jahre, bleibt bis heute die optimale zeitliche Synchronisation des Kontrastmittelbolus in der Zielregion (bolus ti*ming*) und die optimale Nutzung des injizierten Kontrastmittelbolus häufig eine Herausforderung für das Personal, da große Erfahrung und grundlegendes physikalisches Verständnis des komplizierten bildgebenden MRT-Verfahrens gefordert sind.

Insbesondere die in der Gefäßdiagnostik wichtige periphere Angiographie zur Erkennung diabetisch bedingter Gefäßerkrankungen, wie zum Beispiel peripherer arterieller Verschlusskrankheit, kardiovaskulärer Erkrankungen oder "Raucherbein", der unteren Gliedmaßen ist nach wie vor mit einer unerwünscht hohen Rate von artefaktbehafteten und nichtdiagnostischen Untersuchungsergebnissen verbunden.

Insgesamt besteht das Bedürfnis, den zeitlichen Kontrastmittelkonzentrationsverlauf nach der Gabe eines Bolus an einem Punkt im Gefäßsystem eines Patienten möglichst genau vorhersagen zu können, wobei allerdings auf die Verwendung eines vor der Untersuchung applizierten Testbolus verzichtet werden soll.

Es ist daher Aufgabe der Erfindung zur Verbesserung des Verfahrens zur Bestimmung des Kontrastmittelverlaufes an einer vorgegebenen Position des Blutkreislaufes bei der diagnostischen Bildgebung mit einem Magnetresonanzsystem im Blutkreislauf eines Patienten die Vorhersage der Verbreiterung eines Bolus zu ermöglichen, wobei auf die Gabe eines Testbolus beim untersuchten Patienten verzichtet wird.

Diese Aufgabe wird durch die Merkmale der unabhängigen Patentansprüche gelöst. Vorteilhafte Weiterbildungen der Erfindung sind Gegenstand untergeordneter Ansprüche.

Die Erfinder haben erkannt, dass es im Gegensatz zu den zeitlich relativ langen Kontrastmittelgaben bei einer CT-Untersuchung, bei der eine geringe Verbreiterung (Dispersion) des Bolus in Relation zur Dauer der Anflutung kaum eine Rolle spielt, bei der kontrastmittelunterstützten MR-Bildgebung, insbesondere arterieller, Blutgefäße notwendig ist, den Effekt der Verbreiterung einer kurzen Kontrastmittelgabe, insbesondere im Bereich weniger Sekunden zu kennen und zu berücksichtigen, um den Kontrastmittelverlauf möglichst genau vorhersagen zu können. Weiterhin haben die Erfinder erkannt, dass zwischen der Größe der Verbreiterung und den Blutflusseigenschaften, wie beispielsweise der Flussgeschwindigkeit oder dem Volumenfluss, des Blutes im Gefäßsystem am ausgewählten Ort beziehungsweise der Geschwindigkeitsdifferenz zwischen zwei ausgewählten Orten eine auswertbare Korrelation besteht, welche eine Vorhersage einer eintretenden Verbreiterung eines Kontrastmittelbolus erlaubt. Um möglichst aussagekräftige Korrelationswerte zu erhalten, ist es dabei besonders günstig, wenn bei einer Ermittlung der Korrelationswerte auch möglichst vergleichbare Patientenkonstitutionen verwendet werden, also möglichst zum zu untersuchenden Patienten passende Parameter wie Geschlecht, Größe, Gewicht, etc. verwendet werden.

Da es möglich ist, die Flussgeschwindigkeit oder den Blutfluss und andere damit korrelierte Blutflusseigenschaften durch eine Phasenkontrast-Magnetresonanzmessung an vorgegebenen Orten im Gefäßsystem ohne die Anwendung von Kontrastmittel zu bestimmen, eröffnet sich nun die Möglichkeit mit Hilfe einer unmittelbar vor der Untersuchung ausgeführten kontrastmittelfreien Bestimmung der Blutflusseigenschaften an vorgegebenen Gefäßpositionen auf die zu erwartende Verbreiterung des Kontrastmittelbolus rückzuschließen.

Somit kann durch vorhergehende, gegebenenfalls kontrastmittelgestützte, Untersuchungen an vergleichbaren Patientenkollektiven die Korrelation zwischen der Verbreiterung eines applizierten Kontrastmittelbolus und im Gefäßsystem gemessenen Blutflusseigenschaften oder davon abgeleiteten Parametern bestimmt werden. Bei der kontrastmittelgestützten Untersuchung eines Patienten kann dann zunächst unter Abwesenheit von Kontrastmittel die entsprechende Blutflusseigenschaft oder davon abhängige Parameter bestimmt werden und hieraus auf die zu erwartende Verbreiterung des Bolus, zum Beispiel über die Anwendung einer Look up - Tabelle (LUT), geschlossen werden. Da bekannterweise auch die Laufzeit eines Bolus von den vorliegenden Flussgeschwindigkeiten beziehungsweise Volumenflüssen abhängig ist, kann eine solche LUT auch für die Bestimmung der Laufzeit des Bolus (BTT) erstellt werden, oder es kann hier eine andere bekannte Methode zur Vorhersage einer BTT verwendet werden. Aus beiden Informationen kann dann die tatsächlich zu erwartende Anfangs- und Endzeit eines applizierten Bolus vorhergesagt und zur zeitlichen Synchronisierung eines applizierten Bolus mit einer kontrastmittelgestützten MRT-Messung verwendet werden.

Alle hierfür notwendigen Informationen lassen sich in einer aktuellen Untersuchung eines Patienten mit Hilfe einer Phasenkontrast-MRT-Untersuchung ohne die Anwesenheit von Kontrastmittel erheben. Da bei der Messung im Körper des Patienten kein sonstiges vorher appliziertes Kontrastmittel vorhanden ist, welches zu einem erhöhten Hintergrundrauschen also zu einem verschlechterten Signal zu Rausch - Verhältnis und damit zu schlechteren Bildergebnissen führen kann, ergibt sich außerdem eine Verbesserung der Bildqualität. Dies gilt insbesondere deswegen, da sich das erfindungsgemäße Verfahren ausschließlich auf MR-Untersuchungen bezieht, bei denen die MR-Untersuchung im "First Pass", also in der ersten Anflutungsphase des applizierten Kontrastmittels im Bereich des betrachteten ROI, vorgenommen wird und somit bei der Bildgebung keine Kontrastmittel im "Hintergrund" vorliegen.

Das oben beschriebene Verfahren eignet sich besonders zur Darstellung in der Peripherie der unteren Extremitäten (CE-pMRA), denn die aktuellen Limitationen beruhen beim aktuellen Stand der Technik regelmäßig auf einer ungenauen oder unvollständigen Kenntnis des Anwenders der individuellen kardiovaskulären Verhältnisse des Patienten, wie sie unmittelbar vor der Kontrastmitteluntersuchung vorliegen. Durch die unmittelbar vor der bildgebenden Kontrastmitteluntersuchung bestimmten Flussgeschwindigkeiten ergibt sich eine besonders aktuelle, genauere und umfangreichere Kenntnis der individuellen kardiovaskulären Verhältnisse des Patienten als bisher üblich, da auch aktuelle Stresszustände des Patienten in der Untersuchungsumgebung automatisch berücksichtigt werden.

Die bisher praktizierte genaue Kenntnis eines einzelnen skalaren Parameters, wie der Bolusankunftszeit (BAT) in Sekunden nach dem Startzeitpunkt der Kontrastmittelinjektion oder der Bolustransferzeit (BTT), bedeutet zwar eine notwendige aber keine hinreichende Voraussetzung für die diagnostisch optimierte CE-pMRA in allen Untersuchungsfällen. Durch die zusätzliche Vorhersage und Kenntnis der Verbreiterung des Kontrastmittelbolus auf seinem Weg von der i.V. Injektion bis zur Zielregion (ROI) lässt sich nun der zu erwartende Kontrastmittelkonzentrationsverlauf genauer verstehen und bestimmen.

Erfindungsgemäß lassen sich also an verschiedenen Positionen des arteriellen Gefäßsystems Blutflussmessungen durchführen, um aufgrund einer postulierten und erstmals nachgewiesenen Korrelation der Bolusdispersion und verschiedener Blutflussparameter Kenntnis über die akuten und patientenspezifischen kardiovaskulären Verhältnisse zu erlangen, so dass ein notwendiger Kontrastmittelbolus zur Erreichung eines gewünschten Kontrastmittelkonzentrationsprofils und Timings relativ zur Ausführung der MR-Untersuchung mit verbesserter Sicherheit korrekt ausgewählt werden kann.

Demgemäß schlagen die Erfinder ein Verfahren zur Optimierung der Vorbestimmung des zeitlichen Verlaufes einer Kontrastmittelkonzentration an einer Gefäßposition in einer Untersuchungsumgebung bei einer kontrastmittelgestützten MR-Aufnahme eines Patienten ausschließlich in der ersten Anflutungsphase des Kontrastmittels vor, bei dem eine Verbreiterung eines Kontrastmittelbolusverlaufes mit einer ersten Breite an einer vorgegebenen ersten Gefäßposition eines Patienten zu einem Kontrastmittelkonzentrationsverlauf mit einer zweiten Breite an einer vorgegebenen zweiten Gefäßposition, die in der Untersuchungsumgebung angeordnet ist, durch Bestimmung mindestens eines, zumindest von einer Blutflusseigenschaft an einer dritten Gefäßposition abhängigen und mit der Verbreiterung korrelierten, Flussparameters ermittelt wird.

Es wird darauf hingewiesen, dass unter dem Begriff Kontrastmittelkonzentrationsverlauf nicht nur der Verlauf der tatsächlichen Konzentration des Kontrastmittels im Blut, sondern auch der Verlauf der kontrastmittelbedingten Anstiegs des MR-Signals bei einer MR-Untersuchung zu verstehen ist. Weiterhin ist das Verfahren speziell dazu vorgesehen den Kontrastmittelbolusverlauf für Gefäßpositionen zu bestimmen, welche strömungsbezogen hinter der Herz-Lungen-Passage, also arteriell hinter dem Herzen, angeordnet sind, insbesondere sind hierunter periphere Gefäßpositionen und/oder Untersuchungsumgebungen zu verstehen, welche sich in einem peripheren Bereich des Patienten, insbesondere in einer der Gliedmaßen, befinden.

Die ermittelte zu erwartende Verbreiterung zur Vorbestimmung des zeitlichen Kontrastmittelkonzentrationsverlaufes an einer vorgegebenen Gefäßposition kann damit zur weiteren Verwendung zur Verfügung gestellt werden, wobei die Speicherung vorzugsweise in einem elektronischen Speicher zur weiteren Verwendung durch einen Computer ausgeführt wird.

Insbesondere kann die mindestens eine Blutflusseigenschaft unter Abwesenheit von Kontrastmittel durch eine Phasenkontrast-Magnetresonanz-Messung ermittelt werden. Eine solche Messung kann unmittelbar vor der eigentlichen MR-Untersuchung vorgenommen werden, so dass vorteilhaft eine solche Messung die aktuell tatsächlich vorhandene Konstitution und Verfassung der untersuchten Patienten berücksichtigt und damit optimal auf die eigentliche MR-Untersuchung abgestimmt ist.

Grundsätzlich ist es möglich die erfindungsgemäße Bestimmung der Verbreiterung eines Bolus mit einer im Stand der Technik bekannten Bestimmung einer Bolustransferzeit BTT oder Bolusankunftszeit BAT, die gegebenenfalls auch einen Testbolus verwendet, zu kombinieren, um insgesamt einen Kontrastmittelkonzentrationsverlauf vorherzusagen. Es ist jedoch besonders vorteilhaft, wenn auch die zusätzlich bestimmte Bolus-Transfer-Zeit zwischen der ersten Gefäßposition und der zweiten Gefäßposition oder die Bolusankunftszeit aufgrund einer vorbestimmten Korrelation zu einer kontrastmittelfrei gemessenen Blutflusseigenschaft bestimmt wird.

Es wird weiterhin vorgeschlagen, zur Optimierung der Vorbestimmung des zeitlichen Verlaufes einer Kontrastmittelkonzentration an einer Gefäßposition in einer Untersuchungsumgebung bei einer kontrastmittelgestützten MR-Aufnahme eines Patienten die folgenden Verfahrensschritte auszuführen:

- Bestimmung einer Korrelation zwischen einer Verbreiterung mindestens eines an der ersten Gefäßposition vorliegenden Kontrastmittelbolusverlaufes mit einer ersten Breite zum Kontrastmittelkonzentrationsverlauf mit einer zweiten Breite an der zweiten Gefäßposition mit einem, zumindest von einer Blutflusseigenschaft an einer dritten Gefäßposition abhängigen, Flussparameter unter Berücksichtigung mindestens eines Patientenparameter eines untersuchten Patientenkollektivs,
- Bestimmung mindestens eines aktuellen Flussparameters des Patienten,
- Auswahl eines gewünschten Kontrastmittelkonzentrationsverlaufes an der zweiten Gefäßposition und Ermittlung des hierfür notwendigen Kontrastmittelbolusverlaufes aus der zuvor ermittelten Korrelation unter Berücksichtigung mindestens eines Patientenparameters des Patienten,
- Auswahl des notwendigen Kontrastmittelbolusverlaufes an einer Kontrastmittelinjektionsvorrichtung oder Übertragung des notwendigen Kontrastmittelbolusverlaufes an eine Kontrastmittelinjektionsvorrichtung zur Verwendung bei einer kontrastmittelunterstützten MR-Untersuchung, insbesondere einer MR-Angiographie.

Erfindungsgemäß wird auch vorgeschlagen, dass die Bestimmung der Blutflusseigenschaft zumindest an der dritten Gefäßposition des Patienten durch eine Phasenkontrast-Magnetresonanz-Untersuchung ausgeführt wird. Insbesondere kann hierbei die Phasenkontrast-Magnetresonanz-Untersuchung zur Bestimmung der Blutflusseigenschaft unter Abwesenheit von Kontrastmittel im Blutkreislauf des Patienten ausgeführt werden.

Damit für den aktuell zu untersuchenden Patienten auch möglichst auf einen Datenpool aus Voruntersuchungen zurückgegriffen wird, bei dem die dort ermittelte Verbreiterung möglichst charakteristisch für den aktuellen Patienten ist, sollte das Patientenkollektiv, aus dem die Daten der Voruntersuchung bezüglich der Verbreiterung stammen möglichst ähnlich zum aktuell untersuchten Patienten sein. Dies kann dadurch sicher gestellt werden, dass der aktuelle Patient und das relevante Patientenkollektiv aus dem die Daten der Voruntersuchung für die Verbreiterung stammen, bezüglich möglichst vieler Patientenparameter übereinstimmen. Insbesondere sollten dabei Patientenparameter beachtet werden, die einen anatomischen, physiologischen und medizinischen Bezug zum Kreislauf und zur Gefäßstruktur aufweisen. Insbesondere kann demnach als mindestens ein Patientenparameter mindestens eine der folgenden Größen: Geschlecht, Gewicht, Größe, Alter, Herzfrequenz und/oder BMI verwendet werden.

Es wird außerdem auch noch darauf hingewiesen, dass ergänzend zu den o.g. Patientenparametern auch noch das Kontrastmittel selbst als zusätzlicher, die Verbreiterung charakterisierender Parameter eingeführt werden kann. Entsprechend kann die vorgeschlagene LUT um diesen Kontrastmittel-Parameter zur Typisierung des verwendeten Kontrastmittels erweitert werden, ohne den Rahmen der Erfindung zu verlassen.

Bezüglich der betrachteten Blutflusseigenschaften können unterschiedlichste Flusseigenschaften genutzt werden, die eine Korrelation zur Verbreiterung eines Bolus aufweisen. Dabei ist zu beachten, dass bekanntermaßen der Blutfluss durch ein Gefäß in der Regel nicht durch eine einzige über den Gefäßquerschnitt und über die Pulsphase und längere Zeit konstante und unmittelbar messbare Größe definiert werden kann. So charakterisiert sich der Blutfluss durch ein Gefäß an einer Gefäßposition nicht nur durch eine Geschwindigkeit in cm/s oder einen Blutfluss in mL/s sondern sich zeitlich und örtlich ändernde Profile. Trotzdem oder gerade deswegen lassen sich durch entsprechende Messungen charakteristische Eigenschaften des Blutflusses, wie zum Beispiel minimale, maximale oder Durchschnittswerte von Geschwindigkeit und Fluss, ausdrücken, die mit der Verbreiterung eines Bolus in guter Korrelation stehen.

Entsprechend schlagen die Erfinder hierzu vor, dass als mindestens eine Blutflusseigenschaft mindestens eine der nachfolgenden genannten Eigenschaften - wobei jede einzelne der Strichaufzählungen selbst nochmals mehrere wiederum einzeln verwendbare Eigenschaften enthält - verwendet wird:

- maximale, minimale oder mittlere Blutflussgeschwindigkeit an mindestens einer vorgegebenen Position im Gefäßquerschnitt an der Gefäßposition;
- maximale, minimale oder mittlere Blutflussgeschwindigkeit an mindestens einer vorgegebenen Position im Gefäßquerschnitt an der Gefäßposition zu einer vorgegebenen Herz- oder Pulsphase;
- maximale, minimale oder mittlere Blutflussgeschwindigkeit an mindestens einer vorgegebenen Position im Gefäßquerschnitt an der Gefäßposition zu einer vorgegebenen Herz- oder Pulsphase über einen vorgegebenen Messzeitraum;
- maximales, minimales oder mittleres Blutflussvolumen an mindestens einer vorgegebenen Position im Gefäßquerschnitt an der Gefäßposition;
- maximale, minimale oder mittlere Blutflussvolumen an mindestens einer vorgegebenen Position im Gefäßquerschnitt an der Gefäßposition zu einer vorgegebenen Herz- oder Pulsphase;
- maximale, minimale oder mittlere Blutflussvolumen an mindestens einer vorgegebenen Position im Gefäßquerschnitt an der Gefäßposition zu einer vorgegebenen Herz- oder Pulsphase über einen vorgegebenen Messzeitraum;
- eine geometrische Eigenschaft eines Geschwindigkeitsprofils über den Gefäßquerschnitt an der Gefäßposition;
- Nettovorwärtsvolumen über einen vorgegebenen Zeitraum oder pro Herzschlag.

In einer besonders einfachen Ausführungsvariante des erfindungsgemäßen Verfahrens kann als Flussparameter die Blutflusseigenschaft selbst verwendet werden.

Grundsätzlich besteht auch die Möglichkeit die Messung der Blutflusseigenschaften nicht nur an einer Gefäßposition auszuführen, sondern diese Eigenschaften an verschiedenen Gefäßpositionen zu ermitteln und damit eine besonders starke Aussage auch über die Eigenschaften des Weges zwischen den Gefäßpunkten und damit über Gefäßeigenschaften zu erhalten, die besonders mit der Verbreiterung eines Bolus über eine Gefäßstrecke korreliert ist.

Entsprechend wird vorgeschlagen, an mindestens einer vierten Gefäßposition ebenfalls Blutflusseigenschaften zu messen und als Flussparameter eine absolute oder prozentuale Differenz zwischen der Blutflusseigenschaft an der dritten Gefäßposition zur, vorzugsweise gleichen, Blutflusseigenschaft an der mindestens einen vierten Gefäßposition zu verwenden.

Bezüglich besonders günstiger Gefäßpositionen für die auszuführenden Messungen werden von den Erfindern wie folgt vorgeschlagen.

Demgemäß kann die erste Gefäßposition mindestens eine der nachfolgenden Ortsangaben oder Bedingungen erfüllen:

- die erste Gefäßposition liegt in einem venösen Gefäß,
- die erste Gefäßposition liegt in einer Armvene,
- die erste Gefäßposition liegt am Handrücken,
- die erste Gefäßposition liegt an einem zentralvenösen Katheter,
- die erste Gefäßposition liegt zwischen Handrücken und Vena axillari,
- die erste Gefäßposition liegt zwischen Fuß und Vena saphena magna,
- die erste Gefäßposition liegt in einem zentralvenösen Gefäß.

Die zweite Gefäßposition sollte mindestens eine der nachfolgenden Ortsangaben oder Bedingungen erfüllen:

- die zweite Gefäßposition liegt in einem arteriellen Gefäß,
- die zweite Gefäßposition liegt in einer Beinarterie,
- die zweite Gefäßposition liegt flussabwärts zur dritten Gefäßposition,
- die zweite Gefäßposition liegt flussabwärts der Bifurcation,
- die zweite Gefäßposition liegt in einer peripheren Arterie, vorzugsweise im Kniebereich,
- die zweite Gefäßposition liegt im Armbereich,
- die zweite Gefäßposition liegt im Halsbereich.

Bezüglich der dritten Gefäßposition wird vorgeschlagen, dass die dritte Gefäßposition mindestens eine der nachfolgenden Ortsangaben oder Bedingungen erfüllt:

- die dritte Gefäßposition ist die zweite Gefäßposition,
- die dritte Gefäßposition liegt in der Aorta thorakalis,
- die dritte Gefäßposition liegt in der Aorta abdominalis,
- die dritte Gefäßposition liegt zwischen Aorta thorakalis,
- die dritte Gefäßposition liegt vor der Bifurcation,
- die dritte Gefäßposition liegt zwischen Bifurcation und der zweiten Gefäßposition.

Im Falle der Verwendung einer Messung an einer vierten Gefäßposition schlagen die Erfinder vor, dass die vierte Gefäßposition mindestens eine der nachfolgenden Ortsangaben oder Bedingungen erfüllen sollte:

- die vierte Gefäßposition liegt flussaufwärts der dritten Gefäßposition,
- die vierte Gefäßposition liegt flussabwärts zwischen der dritten Gefäßposition und der zweiten Gefäßposition,
- die vierte Gefäßposition liegt flussabwärts der zweiten Gefäßposition.

Das erfindungsgemäße Verfahren betrifft ausschließlich kontrastmittelgestützte MR-Untersuchungen und keine CT-Untersuchungen. Wie bereits zuvor dargestellt ergibt sich daraus die Besonderheit, dass die Zeitspanne einer Bolusgabe besonders kurz sein muss. Daher schlagen die Erfinder bezüglich der Breite des Kontrastmittelbolusverlaufes vor, dass dieser im Bereich von 1 bis 20 Sekunden, vorzugsweise 3 bis 15 Sekunden, liegt. Selbstverständlich bewegt sich die Breite des Kontrastmittelkonzentrationsverlaufes im Gefäßsystem unter Berücksichtigung der jeweils vorliegenden Verbreiterung im ähnlichen Rahmen. Während die Breite des Kontrastmittelbolusverlaufes aufgrund seiner steilen Kanten eindeutig bestimmt ist, unterliegt die Definition der Breite des Kontrastmittelkonzentrationsverlaufes einer gewissen Unschärfe. In der Regel wird der Fachmann daher die Breite als Halbwertsbreite der Kontrastmittelkonzentration oder eines durch das Kontrastmittel erzeugten Signals ansetzen oder das Erreichen eines vorgegebenen Konzentrationswertes oder das Erreichen eines dadurch erzeugten Signalpegels zur Breitendefinition verwenden. Um vergleichbare Aussagen zwischen den Daten aus den Voruntersuchungen und den aktuellen Messdaten bei einer MR-Untersuchung des Patienten zu erreichen, sollte allerdings jeweils die gleiche Breitendefinition verwendet werden. Grundsätzlich kann im Rahmen der Erfindung eine zuvor ermittelte Korrelation zwischen der zu erwartenden Verbreiterung und dem jeweils betrachteten Flussparameter in beliebiger Weise dargestellt werden, beispielsweise auch durch die Verwendung eines mathematisch beschriebenen funktionalen Zusammenhangs. Besonders günstig und einfach ist allerdings die Anwendung einer "look up"-Tabelle (LUT), so dass die Korrelation der Verbreiterung mit dem Flussparameter einer, vorzugsweise elektronisch, abgespeicherten Tabelle entnommen wird. Hierbei kann die aus Referenzmessungen vorbestimmte Tabelle auch mindestens einen der zuvor genannten Patientenparameter enthalten. Alternativ kann die Korrelation der Verbreiterung mit dem Flussparameter durch eine aus Referenzmessungen vorbestimmte Funktion berechnet werden, wobei auch die aus Referenzmessungen vorbestimmte Funktion auch mindestens einen Patientenparameter als Variable enthalten kann.

Neben dem oben beschriebenen Verfahren zählt zum Rahmen der Erfindung auch ein Computer, zumindest aufweisend einen Speicher für Programme, welche im Betrieb ausgeführt werden, wobei dort Programme gespeichert sind, welche das erfindungsgemäße Verfahren gemäß einer hier offenbarten Merkmalskombination ausführen.

Weiterhin zählt zur Erfindung auch ein Magnetresonanzsystem zur Erzeugung einer kontrastmittelunterstützten MR-Aufnahme oder einer MR-Gefäßdarstellung mit einem, von einem Computer gesteuerten Kontrastmittelinjektor, der Computer mit einem Speicher für Programme, die im Betrieb ausgeführt werden, ausgestattet ist, wobei Programme gespeichert sind, welche das erfindungsgemäße Verfahren ausführen.

Außerdem zählt es ebenfalls zum Rahmen der Erfindung, wenn an Stelle der oder ergänzend zur Bestimmung der Bolusverbreiterung in gleicher Weise eine Korrelation zwischen Bolustransferzeit und mindestens einem der hier beschriebenen Flussparameter empirisch und gegebenenfalls patientenparameterabhängig ermittelt und entsprechend abgelegt wird, so dass für die aktuelle Untersuchung eines Patienten auch die BTT durch eine unmittelbar vorhergehende kontrastmittelfreie Phasenkontrast-MR-Untersuchung die Bestimmung mindestens eines benötigten Flussparameters erfolgt und anhand der ermittelten Korrelation die zu erwartende Bolustransferzeit vorausgesagt wird.

Nachfolgend wird die Erfindung anhand der Figuren näher beschrieben, wobei nur die zum Verständnis der Erfindung notwendigen Merkmale dargestellt sind. Es zeigen im Einzelnen:

FIG 1:   Darstellung eines erfindungsgemäßen MRT-Systems;

FIG 2:   Darstellung eines Kreislaufs eines Patienten;

FIG 3:   Darstellung der Veränderung der Breite eines Kontrastmittelbolusverlaufes an einer ersten Gefäßposition zur Breite des Kontrastmittelkonzentrationsverlaufes an einer zweiten Gefäßposition;

FIG 4: Darstellung der gemessenen Signalstärken einer kontrastmittelunterstützten MR-Untersuchung an den Gefäßpositionen Vena cava und Aorta descendens (AD) bei einem Versuchstier (E639, Minischwein) bei einem Fluss von 33 ml/s an der AD;

FIG 5: Darstellung der gemessenen Signalstärken einer kontrastmittelunterstützten MR-Untersuchung an den Gefäßpositionen Vena cava und Aorta descendens (AD) bei einem Versuchstier (E639, Minischwein) bei einem Fluss von 23 ml/s an der AD.

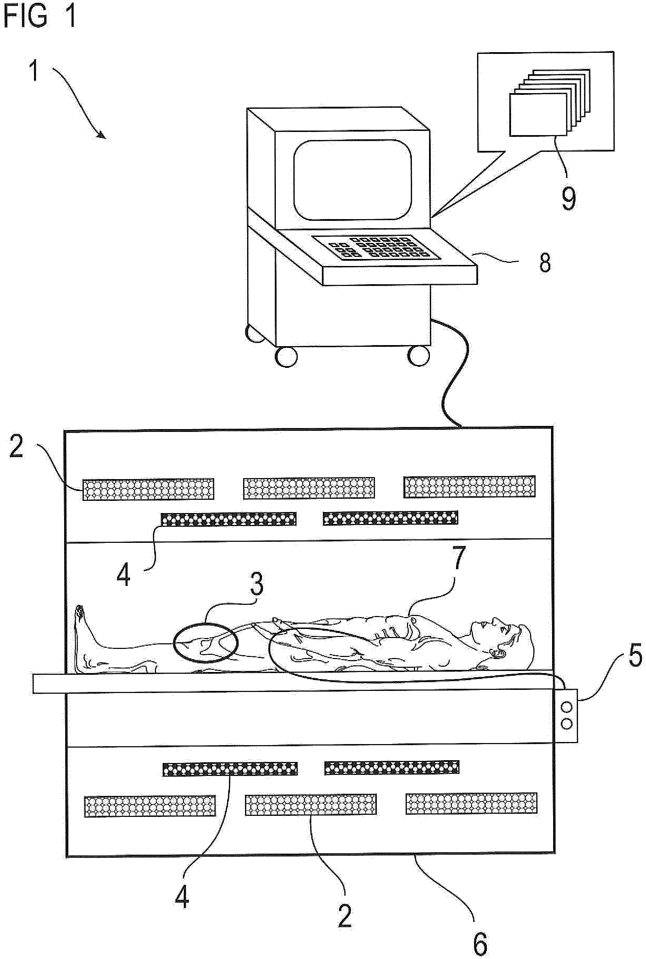

In der **Figur 1** ist schematisch ein Magnetresonanztomographie-System (MRT-System) 1 dargestellt. Bei diesem MRT-System 1 befinden sich in einem Gehäuse 6 Magnetspulen 2 zur Erzeugung eines starken magnetischen Hauptfeldes, wodurch sich die Wasserstoffkerne im Körper des Patienten 7 entsprechend ihrem Spin parallel oder antiparallel zu den Magnetfeldlinien ausrichten. Durch Anregung der Atomkerne mit einem elektromagnetischen Wechselfeld in der Resonanzfrequenz der Atomkerne werden diese zur Schwingung veranlasst. Nach dem Ausschalten der Anregungsfrequenz kehren die Atomkerne wieder in ihre Lage zurück und geben ihre Schwingungsenergie in Form von elektromagnetischer Schwingungsenergie ab, die mit Hilfe von Empfangsspulen 3, welche möglichst in der Nähe einer zu betrachtenden ROI am Patienten 7 angeordnet werden, gemessen wird. Durch zusätzliche Magnetspulen 4 wird ein schwaches Magnetfeld mit einem definierten Feldgradienten erzeugt, wodurch die von den Kernen abgegebenen Signale Ortsinformationen erhalten, durch die die Position des abgegebenen Signals definierbar ist. Die Steuerung dieses Systems 1 und die Auswertung der Mess-Signale erfolgt durch die Steuer- und Recheneinheit 8, welche in ihrem Speicher Programme 9 aufweist, die neben der Steuerung und Bildberechnung auch das erfindungsgemäße Verfahren ausführen.

Zur besseren Darstellung von Blutgefäßen ist es zum Teil notwendig, den Blutkreislauf des Patienten kurzfristig mit Kontrastmittel anzureichern, wozu meist ein Kontrastmittelinjektor 5 verwendet wird, der elektronisch gesteuert - entweder durch die Recheneinheit 8 oder durch eine andere separate Recheneinheit - den Volumenfluss eines zur Messung zu applizierenden Kontrastmittels erzeugt.

Mit Hilfe eines solchen MRT-Systems ist es auch unter Verwendung mehrerer Empfangsantennen möglich ohne die Applikation von Kontrastmittel Blutflussinformationen, wie beispielsweise Flussgeschwindigkeiten, Geschwindigkeitsprofile oder Volumenflüsse, zu erhalten. Diesbezüglich wird lediglich beispielhaft auf die Druckschrift DE 102013204994 A1 verwiesen.

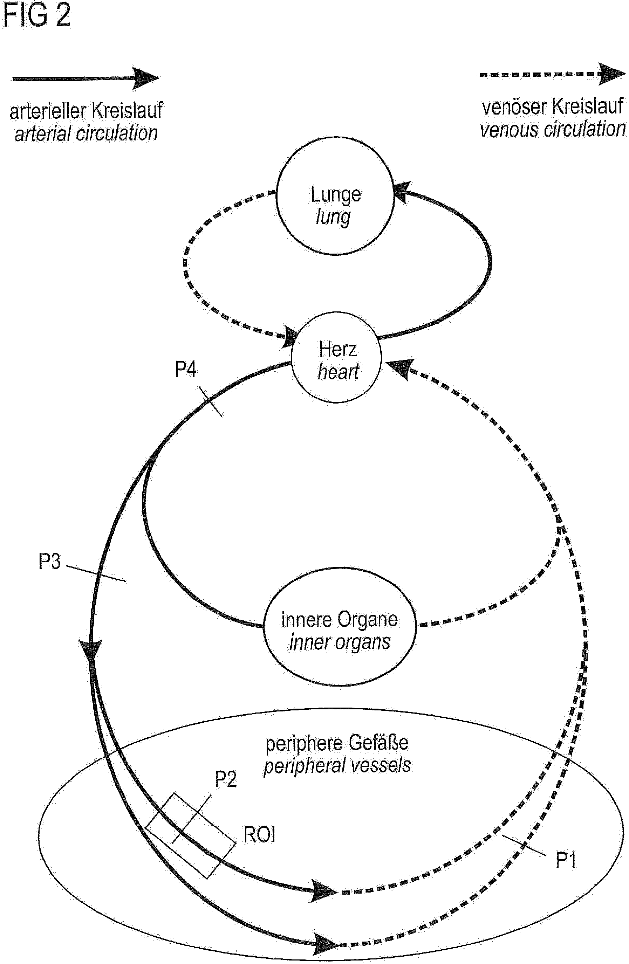

Zum besseren Verständnis der Erfindung wird in der Figur 2 ein sehr schematisiert dargestellter Blutkreislauf eines Patienten gezeigt. Dieser geschlossene Kreislauf teilt sich in einen venösen Kreislauf (gestrichelte Linien) und einen arteriellen Kreislauf (durchgezogene Linien) auf und wird im Wesentlichen durch die Pumptätigkeit des Herzens betrieben. Im Lungenkreislauf ist das arterielle Blut - im Gegensatz zum sonstigen Blutkreislauf - sauerstoffarm und das venöse sauerstoffreich. Entsprechend einem solchen natürlichen Verlauf und den relativ einfach erzeugbaren und nutzbaren Zugängen im venösen Bereich, beispielsweise an einer Arm- oder Handvene, werden üblicherweise Kontrastmittel durch solche Zugänge, meist unter Zuhilfenahme von automatisch gesteuerten Kontrastmittelinjektoren appliziert. Entsprechend passiert ein dort gesetzter Bolus zunächst die nicht näher dargestellten rechten Herzkammern, wird über die Lungenpassage zu den ebenfalls nicht dargestellten linken Kammern des Herzens geleitet und gelangt von dort aus zu den Bereichen des Körpers, die mit einer MR-Aufnahmen abgebildet werden sollen und die bezüglich der Erfindung von Interesse sind, insbesondere auch in die peripheren Gefäßbereiche.

In der schematischen Darstellung der Figur 2 sind die wesentlichen Gefäßpositionen P1 bis P4 und der abzubildende Bereich von Interesse ROI an beispielhaften und typischen Positionen markiert. Wie bereits erwähnt findet die Applikation in den meisten Fällen im Bereich des venösen Kreislaufes, entsprechend der eingetragenen Gefäßposition P1 an einem peripheren Gefäß, zum Beispiel einer Arm- oder Handvene, statt. Das Gebiet von Interesse ROI, das bildgebend untersucht werden soll, kann - wie hier eingezeichnet - beispielsweise im Gebiet einer Beinarterie liegen, in der sich dann auch die Gefäßposition P2 befindet. Die weiteren Messpositionen, an denen dann Blutflusseigenschaften bestimmt werden, liegen dann in der Regel im arteriellen Gefäßsystem zwischen der linken Vorkammer des Herzens und der ROI. Es wird allerdings darauf hingewiesen, dass auch eine Positionierung der Messpunkte an Gefäßpositionen möglich ist, welche in Flussrichtung gesehen hinter der ROI oder P2 angeordnet sind.

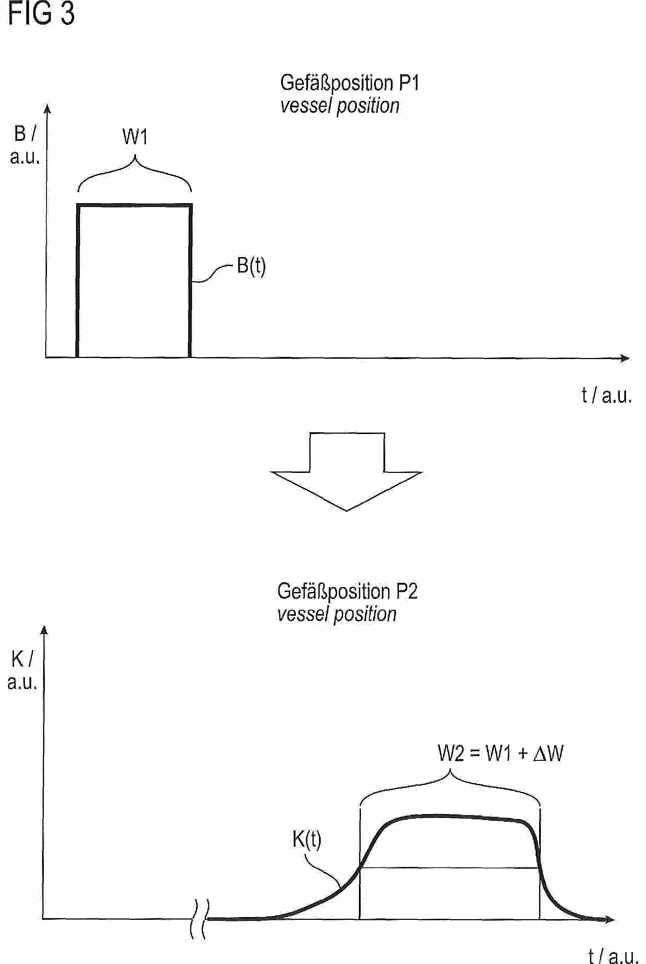

Ein Kontrastmittelbolus, der an einer ersten Gefäßposition P1 appliziert wird hat meist einen Bolusverlauf B(t), wie er in der **Figur 3** im oberen Teil eingezeichnet ist. Dort ist der Volumenfluss eines Bolus B über die Zeit t aufgetragen. Ein solcher Bolusverlauf kann - wie hier dargestellt einen rechteckigen Verlauf annehmen, kann jedoch auch durch entsprechende Einstellung an einem Kontrastmittelinjektor eine beliebige Funktion B(t) nachbilden. Ein solcher Volumenfluss B wird abhängig vom verwendeten Kontrastmittel (z.B. Gadovist® oder Magnevist®) und spezifischen Patienteneigenschaften gewählt, um am Ort der Untersuchung im darzustellenden Gefäß eine gewünschte Kontrastmittelkonzentration zu erreichen. Wie in der Figur 2 gezeigt passiert ein solcher Bolus nach der Applikation im venösen Gefäßbereich zumindest das Herz zweimal und die Lunge mit einer zwischendurch starken Verästelung der Gefäße.

Insbesondere hierdurch bedingt, jedoch auch aufgrund von zusätzlichen Turbulenzen, nicht-laminarer Strömungen und Geschwindigkeitsdifferenzen über den Gefäßquerschnitt und Verdünnungseffekten im venösen System entsteht eine mehr oder minderstarke Dispersion des applizierten Kontrastmittelbolus, was zu einem verbreiterten Kontrastmittelkonzentrationsverlauf K(t) führt. Beispielhaft ist unten in der Figur 3 ein solcher Kontrastmittelkonzentrationsverlauf K(t) an einer Gefäßposition P2 gezeigt. Wie unschwer zu erkennen ist, hat sich die Breite des applizierten Bolus W1 über die zurückgelegte Gefäßstrecke zwischen den Positionen P1 zu P2 wesentlich verbreitert, so dass nun unter Berücksichtigung eine Halbwertsbreite eine Breite von W2 vorliegt. die Verbreiterung ΔW berechnet sich dann aus ΔW = W2-W1.

Erfindungsgemäß wird davon ausgegangen, dass die Größe einer solchen Verbreiterung unmittelbar mit kontrastmittelfrei messbaren Blutflusseigenschaften auf dem Transportweg des Kontrastmittels beziehungsweise daraus ableitbaren Flussparametern in eindeutiger Korrelation steht, so dass durch Ermittelung dieser Flussparameter, insbesondere unter Anwendung von kontrastmittelfreien Messungen von Blutflusseigenschaften, sich die strömungsbedingte Verbreiterung eines applizierten Bolus bestimmen und daher vorhersagen lässt. Unter gleichzeitiger Ermittlung der Bolustransferzeit BTT und/oder Bolusankunftszeit BAT kann dann der zeitliche Verlauf der Kontrastmittelkonzentration an einem Gefäß, insbesondere peripher gelegenen Gefäß, bestimmt und damit aufgrund der Kenntnis des applizierten Bolus vorhergesagt werden. Entsprechend kann damit natürlich auch rückwirkend der notwendige Verlauf einer Bolusapplikation bestimmt werden, der zur Erreichung eines gewünschten Kontrastmittelkonzentrationsverlaufes und damit auch zu einem gewünschten Signalverlauf bei einer MR-Messung an einer ROI führt.

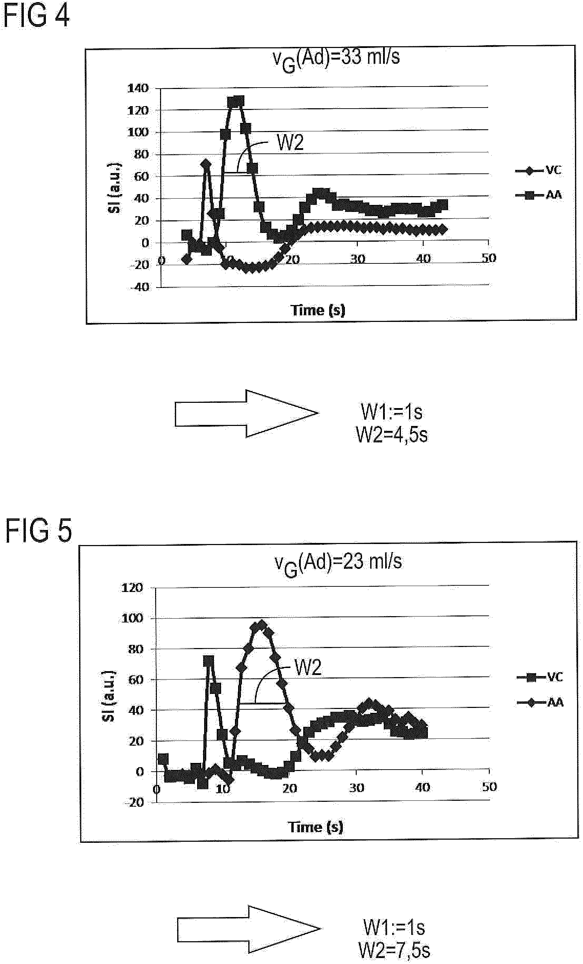

Wie aus den Figuren 4 und 5 hervorgeht, lässt sich eine solche Korrelation zwischen einem einfachen Flussparameter - in Form eines kontrastmittelfrei gemessenen Volumenflusses - und der erzeugten Verbreiterung auch im Tierexperiment - hier mit einem Minischwein - nachweisen.

Die **Figur 4** zeigt - entsprechend einer Bolusapplikationsbreite von W1=1s - einen gemessenen Signalverlauf einer MR-Messung unmittelbar nach der Applikationsstelle (Ohrvene) mit rautenförmigen Messpunkten und einen Signalverlauf einer MR-Messung an der Aorta descendens (AD). Als Blutflusseigenschaft, die direkt auch als Flussparameter verwendet wird, wurde der mittlere Volumenfluss v(AD) in der Aorta descendens bestimmt, der im gezeigten Beispiel 33 ml/s betrug. Die gemessene Breite bezogen auf die Halbwertsbreite des Signalverlaufes beträgt 4,5 Sekunden. Die Verbreiterung bezogen auf die Bolusbreite W1 von 1 Sekunde betrug demnach ΔW = W2-W1 = 4,5s - 1,0s = 3,5s.

Zum Vergleich zeigt die **Figur 5** bei gleicher Bolusapplikationsbreite von W1=1s ebenfalls einen gemessenen Signalverlauf einer MR-Messung unmittelbar nach der Applikationsstelle (Ohrvene) mit rautenförmigen Messpunkten und einen Signalverlauf einer MR-Messung an der Aorta descendens (AD). Der mittlere Volumenfluss v(AD) in der Aorta descendens beträgt bei dieser Messung allerdings 23 ml/s. Die gemessene Breite bezogen auf die Halbwertsbreite des Signalverlaufes beträgt 7,5 Sekunden. Die Verbreiterung bezogen auf die Bolusbreite W1 von 1 Sekunde betrug demnach ΔW = W2-W1 = 7,5s - 1,0s = 6,5s.

Es besteht somit offensichtlich ein korrelierter Zusammenhang zwischen dem gemessenen Blutvolumenfluss v(AD) an der Aorta descendens und der Größe der Verbreiterung ΔW, wie es auch weitere Versuchsergebnisse bestätigen.

Erfindungsgemäß kann ein solcher Zusammenhang in Form einer Look-up-Tabelle (LUT) zur Verfügung gestellt werden, die in einem Computerspeicher abgelegt wird und zur Vorbestimmung der Verbreiterung eines Bolus dienen kann.

Ein Beispiel einer solchen Look-up-Tabelle (LUT) zur Vorbestimmung der Verbreiterung könnte demgemäß wie folgt aussehen:

Tabelle 1:

| W1 \ $P_G$ | $P_G^{-2}$ | $P_G^{-1}$ | $P_G^{avg}$ | $P_G^{+1}$ | $P_G^{+2}$ |
|---|---|---|---|---|---|
| W1$^{-2}$ | $\Delta W(P_G^{-2}, T1^{-2})$ | ... | $\Delta W(P_G^{avg}, T1^{-2})$ | ... | $\Delta W(P_G^{+2}, T1^{+2})$ |
| W1$^{-1}$ | ... | | ... | | ... |
| W1$^{avg}$ | $\Delta W(P_G^{-2}, T1^{avg})$ | ... | $\Delta W(P_G^{avg}, T1^{avg})$ | ... | $\Delta W(P_G^{+2}, T1^{avg})$ |
| W1$^{+1}$ | ... | | ... | | ... |
| W1$^{+2}$ | $\Delta W(P_G^{-2}, T1^{+2})$ | ... | $\Delta W(P_G^{avg}, T1^{+2})$ | ... | $\Delta W(P_G^{+2}, T1^{+2})$ |

Die in der Tabelle 1 dargestellte LUT 1 beschreibt die Bolusverbreiterung *ΔW=W2-W1* als Funktion der Bolus-

applikationsbreite W1 und des Flussparameters $P_G$. Hierin werden mit den zwei Eingabeparametern W1 und $P_G$ in Abhängigkeit der Bolusapplikationsbreite W1 und eines in diesem Beispiel einzigen gemessenen Parameters $P_G$ die empirisch vorbestimmte und dazu korrelierte Bolusverbreiterung $\Delta W$ zur Verfügung gestellt. Der Flussparameter $P_G$ kann zum Beispiel den Blutvolumenfluss, gemessen an einer Gefäßposition P3 = Aorta descendens v(AD), darstellen. Die hochgestellten Indizes bezeichnen die unterschiedlichen Wertebereiche der Parameter im Zeilen- beziehungsweise Spaltentitel. Zwischenwerte können gegebenenfalls durch Interpolation gewonnen werden.

Ergänzend oder auch unabhängig kann in gleicher Art auch die Korrelation zwischen, vorzugsweise kontrastmittelfrei, gemessenen Flussparametern und der BTT beziehungsweise BAT bestimmt und dargestellt werden. Eine entsprechende LUT kann demnach wie folgt gestaltet sein:

Tabelle 2:

| BTT von P1 nach P2 | $P_G^{-2}$ | $P_G^{-1}$ | $P_G^{avg}$ | $P_G^{+1}$ | $P_G^{+2}$ |
|---|---|---|---|---|---|
| $d^{-2}(ROI)$ / cm | $BTT(d^{-2}(ROI), P_G^{-2})$ | ... | $BTT(d^{-2}(ROI), P_G^{avg})$ | ... | $BTT(d^{-2}(ROI), P_G^{+2})$ |
| $d^{-1}(ROI)$/ cm | ... | | ... | | ... |
| $d^{avg}(ROI)$ / cm | $BTT(d^{avg}(ROI), P_G^{-2})$ | | $BTT(d^{avg}(ROI), P_G^{avg})$ | | $BTT(d^{avg}(ROI), P_G^{+2})$ |
| $d^{+1}(ROI)$/cm | ... | | ... | | ... |
| $d^{+2}(ROI)$/cm | $BTT(d^{+2}(ROI), P_G^{-2})$ | ... | $BTT(d^{+2}(ROI), P_G^{avg})$ | ... | $BTT(d^{+2}(ROI), P_G^{+2})$ |

In der Tabelle 2 wird eine beispielhafte LUT 2 gezeigt, in der die Werte für die Bolus Travel Time $(BTT = \dfrac{d(ROI)}{v_G(AD)} r_{fl})$ Funktion der Distanz d(ROI) einer ausgewählten typischen Gefäßposition zum ROI und einem ermittelten Flussparameter $P_G$ aus gemessenen spezifischen Blutflusseigenschaften $v_G(Ap)$ und $v_G(Ab)$ mit $P_G = v_G(Ap)/v_G(Ab)$ abgelesen werden können, wobei sich grundsätzlich die BTT aus der Flussgeschwindigkeit $v_G(AD)$ in der Aorta descendens, der zurückzulegenden Distanz d(ROI) und einem flussparameterabhängigen Korrekturfaktor $r_{fl} = \dfrac{v_G(Ap)}{v_G(Ab)}$ ableitet und die Flussgeschwindigkeit der Gleichung $v_G = \dfrac{d}{BTT}$ folgt. Die hochgestellten Indizes bezeichnen jeweils die unterschiedlichen Wertebereiche des jeweiligen Parameters im Zeilen- beziehungsweise Spaltentitel. Zwischenwerte können gegebenenfalls durch Interpolation gewonnen werden.

In dieser LUT 2 werden zur Bestimmung einer Bolus Travel Time (BTT) als Eingabe-Parameter sowohl spezifische Patientenparameter, als auch aktuelle Flussparameter verwendet: ein Patientenparameter ist in Form einer Distanz $d^i(ROI)$ angegeben, die hier den Abstand in cm zwischen einer Gefäßposition P3, an der der Flussparameter $r_{f1}$ gemessen wird, und einer Gefäßposition P2 im Bereich der Zielregion ROI darstellt. Des Weiteren sind als Eingabe-Parameter die aktuellen Flussparameter Blutflussgeschwindigkeiten $V_G(AD)$, $V_G(AB)$ und $V_G(AP)$ an verschiedenen dargestellt. Die Blutflussgeschwindigkeiten $V_G(Ap)$, exemplarisch darstellend die Blutflussgeschwindigkeit im Bereich der Aorta poplitealis, und die Blutflussgeschwindigkeit $V_G(Ab)$, exemplarisch darstellend die Blutflussgeschwindigkeit im Bereich der Bifurcation, gehen dabei als Quotient und Korrekturfaktor in die LUT ein und können damit Patienten-spezifische, die mittlere Flussgeschwindigkeit und damit BTT bestimmende Faktoren, abbilden. Die Blutflussgeschwindigkeit $V_G(AD)$ stellt wieder exemplarisch die im Bereich der Aorta descendens gemessene Blutflussgeschwindigkeit dar.

Durch das Zusammenwirken einer entsprechend angepassten LUT 1 und LUT 2 kann somit das Timing einer Kontrastmittelbolusgabe mit der kontrastmittelfreien Bestimmung verschiedener Blutfluss- und Patientenparameter bestimmt werden.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen. Insbesondere beschränkt sich die Erfindung nicht auf die hier angegebenen Merkmalskombinationen, sondern es können auch für den Fachmann offensichtlich ausführbare andere Kombinationen und Teilkombination aus den offenbarten Merkmalen gebildet werden. Des Weiteren beschränkt sich die Erfindung nicht auf die in den Ansprüchen verwendeten Anspruchskategorien, sondern umfasst auch die offenbarten Merkmale in Verbindung mit allen weiteren Anspruchskategorien.

Insgesamt wird mit der Erfindung also ein Verfahren vorgeschlagen, bei dem zur Optimierung der Vorbestimmung des zeitlichen Verlaufes einer Kontrastmittelkonzentration an einer Gefäßposition in einer Untersuchungsumgebung (ROI) bei einer kontrastmittelgestützten MR-Aufnahme eines Patienten (P) ausschließlich in der ersten Anflutungsphase des Kontrastmittels, die Korrelation einer Verbreiterung und/oder einer Bolustransferzeit eines Kontrastmittelbolus zwischen zwei Gefäßpositionen und mindestens einem Flussparameter - der eine Funktion von mindestens einer Flusseigenschaft darstellt - im Blutkreislauf eines Patientenkollektivs vorbestimmt wird, so dass anschließend vor einer auszuführenden MR-Untersuchung eines Patienten diese Flussparameter durch eine kontrastmittelfreie Phasenkontrast-MR-Untersuchung bestimmt werden und über die vorbekannten Korrelationen zur Verbreiterung und/oder Bolustransferzeit der zu erwartende Kontrastmittelkonzentrationsverlauf an einer Gefäßposition im Untersuchungsbereich bestimmt wird.

Bezugszeichenliste

| | |
|---|---|
| 1 | Magnetresonanztomographie-System (MRT-System) |
| 2 | Magnetspulen |
| 3 | Empfangsspule |
| 4 | Magnetspulen |
| 5 | Kontrastmittelinjektor |
| 6 | Gehäuse |
| 7 | Patient |
| 8 | Steuer- und Recheneinheit / Computer mit Speicher und Display |
| 9 | Computer-Programme |
| AA | Aorta ascendens |

| B | Bolus |
| B(t) | zeitlicher Bolusverlauf |
| K | Kontrastmittelkonzentration |
| K(t) | zeitlicher Kontrastmittelkonzentrationsverlauf |
| P1 | erste Gefäßposition |
| P2 | zweite Gefäßposition |
| P3 | dritte Gefäßposition |
| P4 | vierte Gefäßposition |
| ROI | Untersuchungsgebiet / Gebiet von Interesse |
| SI | Signal |
| t | Zeit |
| VC | Vena Cava |
| $v_G(Ad)$ | Blutflussgeschwindigkeit in der Aorta descendens |
| W1 | Bolusbreite = Bolusdauer |
| W2 | Breite = Dauer des Kontrastmittelkonzentrationsverlaufes |
| $\Delta W$ | Verbreiterung |

**Patentansprüche**

1. Verfahren zur Optimierung der Vorbestimmung des zeitlichen Verlaufes einer Kontrastmittelkonzentration an einer Gefäßposition in einer Untersuchungsumgebung (ROI) bei einer kontrastmittelgestützten MR-Aufnahme eines Patienten (P) ausschließlich in der ersten Anflutungsphase des Kontrastmittels, **dadurch g e kennzeichnet**, dass eine Verbreiterung ($\Delta W$=W2-W1) eines Kontrastmittelbolusverlaufes (B(t)) mit einer ersten Breite (W1) an einer vorgegebenen ersten Gefäßposition (P1) eines Patienten (P) zu einem Kontrastmittelkonzentrationsverlauf (K(t)) mit einer zweiten Breite (W2) an einer vorgegebenen zweiten Gefäßposition (P2), die in der Untersuchungsumgebung (ROI) angeordnet ist, durch Bestimmung mindestens eines, zumindest von einer Blutflusseigenschaft ($f_B$) an einer dritten Gefäßposition (P3) abhängigen und mit der Verbreiterung korrelierten, Flussparameters ($P_G$) ermittelt wird .

2. Verfahren gemäß dem voranstehenden Anspruch 1, **dadurch gekennzeichnet, dass** die zweite Gefäßposition (P2) und/oder die Untersuchungsumgebung (ROI) sich strömungsbezogen hinter der Herz-Lungen-Passage befindet.

3. Verfahren gemäß dem voranstehenden Anspruch 2, **dadurch gekennzeichnet, dass** die zweite Gefäßposition (P2) und/oder die Untersuchungsumgebung (ROI) sich in einem peripheren Bereich des Patienten, insbesondere einer der Gliedmaßen, befinden.

4. Verfahren gemäß einem der voranstehenden Ansprüche 1 bis 3, **dadurch gekennzeichnet, dass** die ermittelte zu erwartende Verbreiterung ($\Delta W$=W2-W1) zur Vorbestimmung des zeitlichen Kontrastmittelkonzentrationsverlauf (K(t)) zur Verfügung gestellt, vorzugsweise in einem elektronischen Speicher zur weiteren Verwendung durch einen Computer abgespeichert, wird.

5. Verfahren gemäß einem der voranstehenden Ansprüche 1 bis 4, **dadurch gekennzeichnet, dass** die mindestens eine Blutflusseigenschaft ($f_B$) unter Abwesenheit von Kontrastmittel durch eine Phasenkontrast-Magnetresonanz-Messung ermittelt wird.

6. Verfahren gemäß einem der voranstehenden Ansprüche 1 bis 5, **dadurch gekennzeichnet, dass** zusätzlich eine Bolus-Transfer-Zeit (BTT) und/oder Bolusankunftszeit (BAT) zwischen der ersten Gefäßposition (P1) und der zweiten Gefäßposition (P2) bestimmt wird.

7. Verfahren gemäß einem der voranstehenden Ansprüche 1 bis 6, **dadurch gekennzeichnet, dass** aus der erwarteten Verbreiterung ($\Delta W$=W2-W1) und einer Bolus-Transfer-Zeit (BTT) und/oder Bolusankunftszeit (BAT) der Kontrastmittelkonzentrationsverlauf (K(t)) an der zweiten Gefäßposition (P2) bestimmt wird.

8. Verfahren zur Optimierung der Vorbestimmung des zeitlichen Verlaufes einer Kontrastmittelkonzentration an einer Gefäßposition in einer Untersuchungsumgebung (ROI) bei einer kontrastmittelgestützten MR-Aufnahme eines Patienten (P), insbesondere gemäß einem der voranstehenden Ansprüche 1 bis 7, aufweisend die folgenden Verfahrensschritte:

8.1. Bestimmung einer Korrelation zwischen einer Verbreiterung ($\Delta W = W2-W1$) mindestens eines an der ersten Gefäßposition (P1) vorliegenden Kontrastmittelbolusverlaufes (B(t)) mit einer ersten Breite (W1) zum Kontrastmittelkonzentrationsverlauf (K(t)) mit einer zweiten Breite (W2) an der zweiten Gefäßposition (P2) mit einem, zumindest von einer Blutflusseigenschaft ($f_B$) an einer dritten Gefäßposition (P3) abhängigen, Flussparameter ($P_G$) unter Berücksichtigung mindestens eines Patientenparameter ($P_P$) eines untersuchten Patientenkollektivs,

8.2. Bestimmung mindestens eines aktuellen Flussparameters ($P_{Pa}$) des Patienten (P),

8.3. Auswahl eines gewünschten Kontrastmittelkonzentrationsverlaufes (K(t)) an der zweiten Gefäßposition (P2) und Ermittlung des hierfür notwendigen Kontrastmittelbolusverlaufes (B(t)) aus der zuvor ermittelten Korrelation unter Berücksichtigung mindestens eines Patientenparameters ($P_P$) des Patienten (P),

8.4. Auswahl des notwendigen Kontrastmittelbolusverlaufes (B(t)) an einer Kontrastmittelinjektionsvorrichtung oder Übertragung des notwendigen Kontrastmittelbolusverlaufes (B(t)) an eine Kontrastmittelinjektionsvorrichtung zur Verwendung bei einer kontrastmittelunterstützten MR-Untersuchung, insbesondere einer MR-Angiographie.

9. Verfahren gemäß dem voranstehenden Patentanspruch 8, **dadurch gekennzeichnet, dass** die Bestimmung der Blutflusseigenschaft ($f_B$) zumindest an der dritten Gefäßposition (P3) des Patienten (P) durch eine Phasenkontrast-Magnetresonanz-Untersuchung ausgeführt wird.

10. Verfahren gemäß dem voranstehenden Patentanspruch 9, **dadurch gekennzeichnet, dass** die Phasenkontrast-Magnetresonanz-Untersuchung zur Bestimmung der Blutflusseigenschaft ($f_B$) unter Abwesenheit von Kontrastmittel im Blutkreislauf des Patienten ausgeführt wird.

11. Verfahren gemäß einem der voranstehenden Patentansprüche 1 bis 10, **dadurch gekennzeichnet, dass** als mindestens ein Patientenparameter ($P_P$) mindestens eine der folgenden Größen verwendet wird:

- Geschlecht,
- Gewicht,
- Größe,
- Alter,
- Herzfrequenz,
- BMI,
- Typisierung der Statur,
- Abstand zwischen vorgegebenen Gefäßpositionen.

12. Verfahren gemäß einem der voranstehenden Patentansprüche 1 bis 11, **dadurch gekennzeichnet, dass** als mindestens eine Blutflusseigenschaft ($f_B$) mindestens eine der folgenden Eigenschaften verwendet wird:

- maximale, minimale oder mittlere Blutflussgeschwindigkeit ($v_G$) an mindestens einer vorgegebenen Position im Gefäßquerschnitt an der Gefäßposition;
- maximale, minimale oder mittlere Blutflussgeschwindigkeit ($v_G$) an mindestens einer vorgegebenen Position im Gefäßquerschnitt an der Gefäßposition zu einer vorgegebenen Herz- oder Pulsphase;
- maximale, minimale oder mittlere Blutflussgeschwindigkeit ($v_G$) an mindestens einer vorgegebenen Position im Gefäßquerschnitt an der Gefäßposition zu einer vorgegebenen Herz- oder Pulsphase über einen vorgegebenen Messzeitraum;
- maximales, minimales oder mittleres Blutflussvolumen an mindestens einer vorgegebenen Position im Gefäßquerschnitt an der Gefäßposition;
- maximale, minimale oder mittlere Blutflussvolumen an mindestens einer vorgegebenen Position im Gefäßquerschnitt an der Gefäßposition zu einer vorgegebenen Herz- oder Pulsphase;
- maximale, minimale oder mittlere Blutflussvolumen an mindestens einer vorgegebenen Position im Gefäßquerschnitt an der Gefäßposition zu einer vorgegebenen Herz- oder Pulsphase über einen vorgegebenen Messzeitraum;
- eine geometrische Eigenschaft eines Geschwindigkeitsprofils über den Gefäßquerschnitt an der Gefäßposition;
- Nettovorwärtsvolumen über einen vorgegebenen Zeitraum oder pro Herzschlag.

13. Verfahren gemäß einem der voranstehenden Patentansprüche 1 bis 12, **dadurch gekennzeichnet, dass** als Flussparameter ($P_G$) die Blutflusseigenschaft ($f_B$) selbst verwendet wird

14. Verfahren gemäß einem der voranstehenden Patentansprüche 1 bis 12, **dadurch gekennzeichnet, dass** als Flussparameter ($P_G$) eine absolute oder prozentuale Differenz zwischen der Blutflusseigenschaft ($f_B$) an der dritten Gefäßposition (P3) zur gleichen Blutflusseigenschaft ($f_B$) an einer vierten Gefäßposition (P4) verwendet wird.

15. Verfahren gemäß einem der voranstehenden Patentansprüche 1 bis 14, **dadurch gekennzeichnet, dass** die erste Gefäßposition (P1) mindestens eine der nachfolgenden Ortsangaben oder Bedingungen erfüllt:

- die erste Gefäßposition (P1) liegt in einem venösen Gefäß,
- die erste Gefäßposition (P1) liegt in einer Armvene,
- die erste Gefäßposition (P1) liegt am Handrücken,
- die erste Gefäßposition (P1) liegt an einem zentralvenösen Katheter,
- die erste Gefäßposition (P1) liegt zwischen Handrücken und V. axillari,
- die erste Gefäßposition (P1) liegt zwischen Fuß und V. saphena magna
- die erste Gefäßposition (P1) liegt in einem zentralvenösen Gefäß.

16. Verfahren gemäß einem der voranstehenden Patentansprüche 1 bis 15, **dadurch gekennzeichnet, dass** die zweite Gefäßposition (P2) mindestens eine der nachfolgenden Ortsangaben oder Bedingungen erfüllt:

- die zweite Gefäßposition (P2) liegt in einem arteriellen Gefäß,
- die zweite Gefäßposition (P2) liegt in einer Beinarterie,
- die zweite Gefäßposition (P2) liegt flussabwärts zur dritten Gefäßposition (P3),
- die zweite Gefäßposition (P2) liegt flussabwärts der Bifurcation,
- die zweite Gefäßposition (P2) liegt in einer peripheren Arterie, vorzugsweise im Kniebereich,
- die zweite Gefäßposition (P2) liegt im Armbereich,
- die zweite Gefäßposition (P2) liegt im Halsbereich.

17. Verfahren gemäß einem der voranstehenden Patentansprüche 1 bis 16, **dadurch gekennzeichnet, dass** die dritte Gefäßposition (P3) mindestens eine der nachfolgenden Ortsangaben oder Bedingungen erfüllt:

- die dritte Gefäßposition (P3) ist die zweite Gefäßposition (P2),
- die dritte Gefäßposition (P3) liegt in der Aorta thorakalis,
- die dritte Gefäßposition (P3) liegt in der Aorta abdominalis,
- die dritte Gefäßposition (P3) liegt zwischen Aorta thorakalis und (P2)
- die dritte Gefäßposition (P3) liegt vor der Bifurcation,
- die dritte Gefäßposition (P3) liegt zwischen Bifurcation und (P2).

18. Verfahren gemäß einem der voranstehenden Patentansprüche 14 bis 17, **dadurch gekennzeichnet, dass** die vierte Gefäßposition (P4) mindestens eine der nachfolgenden Ortsangaben oder Bedingungen erfüllt:

- die vierte Gefäßposition (P4) liegt flussaufwärts der dritten Gefäßposition (P3),
- die vierte Gefäßposition (P4) liegt flussabwärts zwischen der dritten Gefäßposition (P3) und der zweiten Gefäßposition (P2),
- die vierte Gefäßposition (P4) liegt flussabwärts der zweiten Gefäßposition (P2).

19. Verfahren gemäß einem der voranstehenden Patentansprüche 1 bis 18, **dadurch gekennzeichnet, dass** die Breite des Kontrastmittelbolusverlaufes (B(t)) im Bereich von 1 bis 20 Sekunden, vorzugsweise 3 bis 15 Sekunden, liegt.

20. Verfahren gemäß einem der voranstehenden Patentansprüche 1 bis 19, **dadurch gekennzeichnet, dass** die Korrelation der Verbreiterung ($\Delta W = W2 - W1$) mit dem Flussparameter ($P_G$) einer gespeicherten Tabelle (LUT) entnommen wird.

21. Verfahren gemäß dem voranstehenden Patentanspruch 20, **dadurch gekennzeichnet, dass** die aus Referenzmessungen vorbestimmte Tabelle (LUT) auch mindestens einen Patientenparameter ($P_P$) aus der nachfolgenden Liste enthält.

22. Verfahren gemäß einem der voranstehenden Patentansprüche 1 bis 21, **dadurch gekennzeichnet, dass** die Korrelation der Verbreiterung ($\Delta W = W2 - W1$) mit dem Flussparameter ($P_G$) durch eine aus Referenzmessungen vorbestimmte Funktion ($f(P_G, P_P,)$) berechnet wird.

**23.** Verfahren gemäß dem voranstehenden Patentanspruch 22, **dadurch gekennzeichnet, dass** die aus Referenzmessungen vorbestimmte Funktion (f($P_G$, $P_P$)) auch mindestens einen Patientenparameter ($P_P$) als Variable enthält.

**24.** Magnetresonanzsystem zur Erzeugung einer kontrastmittelunterstützten MR-Aufnahme oder einer MR-Gefäßdarstellung mit einem, von einem Computer gesteuerten Kontrastmittelinjektor, der Computer mit einem Speicher für Programme, die im Betrieb ausgeführt werden, ausgestattet ist, **dadurch gekennzeichnet, dass** Programme gespeichert sind, welche das Verfahren gemäß einem der voranstehenden Verfahrensansprüche ausführen.

**Geänderte Patentansprüche gemäss Regel 137(2) EPÜ.**

**1.** Verfahren zur Optimierung der Vorbestimmung des zeitlichen Verlaufes einer Kontrastmittelkonzentration an einer Gefäßposition in einer Untersuchungsumgebung (ROI) bei einer kontrastmittelgestützten MR-Aufnahme eines Patienten (P) ausschließlich in der ersten Anflutungsphase des Kontrastmittels, **dadurch gekennzeichnet, dass** eine Verbreiterung ($\Delta W=W2-W1$) eines Kontrastmittelbolusverlaufes (B(t)) mit einer ersten Breite (W1) an einer vorgegebenen ersten Gefäßposition (P1) eines Patienten (P) zu einem Kontrastmittelkonzentrationsverlauf (K(t)) mit einer zweiten Breite (W2) an einer vorgegebenen zweiten Gefäßposition (P2), die in der Untersuchungsumgebung (ROI) angeordnet ist, durch Bestimmung mindestens eines, zumindest von einer Blutflusseigenschaft ($f_B$) an einer dritten Gefäßposition (P3) abhängigen und mit der Verbreiterung korrelierten, Flussparameters ($P_G$) ermittelt wird .

**2.** Verfahren gemäß dem voranstehenden Anspruch 1, **dadurch gekennzeichnet, dass** die zweite Gefäßposition (P2) und/oder die Untersuchungsumgebung (ROI) sich strömungsbezogen hinter der Herz-Lungen-Passage befindet.

**3.** Verfahren gemäß dem voranstehenden Anspruch 2, **dadurch gekennzeichnet, dass** die zweite Gefäßposition (P2) und/oder die Untersuchungsumgebung (ROI) sich in einem peripheren Bereich des Patienten, insbesondere einer der Gliedmaßen, befinden.

**4.** Verfahren gemäß einem der voranstehenden Ansprüche 1 bis 3, **dadurch gekennzeichnet, dass** die ermittelte zu erwartende Verbreiterung ($\Delta W=W2-W1$) zur Vorbestimmung des zeitlichen Kontrastmittelkonzentrationsverlauf (K(t)) zur Verfügung gestellt, vorzugsweise in einem elektronischen Speicher zur weiteren Verwendung durch einen Computer abgespeichert, wird.

**5.** Verfahren gemäß einem der voranstehenden Ansprüche 1 bis 4, **dadurch gekennzeichnet, dass** die mindestens eine Blutflusseigenschaft ($f_B$) unter Abwesenheit von Kontrastmittel durch eine Phasenkontrast-Magnetresonanz-Messung ermittelt wird.

**6.** Verfahren gemäß einem der voranstehenden Ansprüche 1 bis 5, **dadurch gekennzeichnet, dass** zusätzlich eine Bolus-Transfer-Zeit (BTT) und/oder Bolusankunftszeit (BAT) zwischen der ersten Gefäßposition (P1) und der zweiten Gefäßposition (P2) bestimmt wird.

**7.** Verfahren gemäß einem der voranstehenden Ansprüche 1 bis 6, **dadurch gekennzeichnet, dass** aus der erwarteten Verbreiterung ($\Delta W=W2-W1$) und einer Bolus-Transfer-Zeit (BTT) und/oder Bolusankunftszeit (BAT) der Kontrastmittelkonzentrationsverlauf (K(t)) an der zweiten Gefäßposition (P2) bestimmt wird.

**8.** Verfahren zur Optimierung der Vorbestimmung des zeitlichen Verlaufes einer Kontrastmittelkonzentration an einer Gefäßposition in einer Untersuchungsumgebung (ROI) bei einer kontrastmittelgestützten MR-Aufnahme eines Patienten (P), insbesondere gemäß einem der voranstehenden Ansprüche 1 bis 7, aufweisend die folgenden Verfahrensschritte:

8.1. Bestimmung einer Korrelation zwischen einer Verbreiterung ($\Delta W=W2-W1$) mindestens eines an der ersten Gefäßposition (P1) vorliegenden Kontrastmittelbolusverlaufes (B(t)) mit einer ersten Breite (W1) zum Kontrastmittelkonzentrationsverlauf (K(t)) mit einer zweiten Breite (W2) an der zweiten Gefäßposition (P2) mit einem, zumindest von einer Blutflusseigenschaft ($f_B$) an einer dritten Gefäßposition (P3) abhängigen, Flussparameter ($P_G$) unter Berücksichtigung mindestens eines Patientenparameter ($P_P$) eines untersuchten Patientenkollektivs,
8.2. Bestimmung mindestens eines aktuellen Flussparameters ($P_{Pa}$) des Patienten (P),
8.3. Auswahl eines gewünschten Kontrastmittelkonzentrationsverlaufes (K(t)) an der zweiten Gefäßposition (P2) und Ermittlung des hierfür notwendigen Kontrastmittelbolusverlaufes (B(t)) aus der zuvor ermittelten Korrelation unter Berücksichtigung mindestens eines Patientenparameters ($P_P$) des Patienten (P),

8.4. Auswahl des notwendigen Kontrastmittelbolusverlaufes (B(t)) an einer Kontrastmittelinjektionsvorrichtung oder Übertragung des notwendigen Kontrastmittelbolusverlaufes (B(t)) an eine Kontrastmittelinjektionsvorrichtung zur Verwendung bei einer kontrastmittelunterstützten MR-Untersuchung, insbesondere einer MR-Angiographie.

**9.** Verfahren gemäß dem voranstehenden Patentanspruch 8, **dadurch gekennzeichnet, dass** die Bestimmung der Blutflusseigenschaft ($f_B$) zumindest an der dritten Gefäßposition (P3) des Patienten (P) durch eine Phasenkontrast-Magnetresonanz-Untersuchung ausgeführt wird.

**10.** Verfahren gemäß dem voranstehenden Patentanspruch 9, **dadurch gekennzeichnet, dass** die Phasenkontrast-Magnetresonanz-Untersuchung zur Bestimmung der Blutflusseigenschaft ($f_B$) unter Abwesenheit von Kontrastmittel im Blutkreislauf des Patienten ausgeführt wird.

**11.** Verfahren gemäß einem der voranstehenden Patentansprüche 1 bis 10, **dadurch gekennzeichnet, dass** als mindestens ein Patientenparameter ($P_P$) mindestens eine der folgenden Größen verwendet wird:

- Geschlecht,
- Gewicht,
- Größe,
- Alter,
- Herzfrequenz,
- BMI,
- Typisierung der Statur,
- Abstand zwischen vorgegebenen Gefäßpositionen.

**12.** Verfahren gemäß einem der voranstehenden Patentansprüche 1 bis 11, **dadurch gekennzeichnet, dass** als mindestens eine Blutflusseigenschaft ($f_B$) mindestens eine der folgenden Eigenschaften verwendet wird:

- maximale, minimale oder mittlere Blutflussgeschwindigkeit ($v_G$) an mindestens einer vorgegebenen Position im Gefäßquerschnitt an der Gefäßposition;
- maximale, minimale oder mittlere Blutflussgeschwindigkeit ($v_G$) an mindestens einer vorgegebenen Position im Gefäßquerschnitt an der Gefäßposition zu einer vorgegebenen Herz- oder Pulsphase;
- maximale, minimale oder mittlere Blutflussgeschwindigkeit ($v_G$) an mindestens einer vorgegebenen Position im Gefäßquerschnitt an der Gefäßposition zu einer vorgegebenen Herz- oder Pulsphase über einen vorgegebenen Messzeitraum;
- maximales, minimales oder mittleres Blutflussvolumen an mindestens einer vorgegebenen Position im Gefäßquerschnitt an der Gefäßposition;
- maximale, minimale oder mittlere Blutflussvolumen an mindestens einer vorgegebenen Position im Gefäßquerschnitt an der Gefäßposition zu einer vorgegebenen Herz- oder Pulsphase;
- maximale, minimale oder mittlere Blutflussvolumen an mindestens einer vorgegebenen Position im Gefäßquerschnitt an der Gefäßposition zu einer vorgegebenen Herz- oder Pulsphase über einen vorgegebenen Messzeitraum;
- eine geometrische Eigenschaft eines Geschwindigkeitsprofils über den Gefäßquerschnitt an der Gefäßposition;
- Nettovorwärtsvolumen über einen vorgegebenen Zeitraum oder pro Herzschlag.

**13.** Verfahren gemäß einem der voranstehenden Patentansprüche 1 bis 12, **dadurch gekennzeichnet, dass** als Flussparameter ($P_G$) die Blutflusseigenschaft ($f_B$) selbst verwendet wird

**14.** Verfahren gemäß einem der voranstehenden Patentansprüche 1 bis 12, **dadurch gekennzeichnet, dass** als Flussparameter ($P_G$) eine absolute oder prozentuale Differenz zwischen der Blutflusseigenschaft ($f_B$) an der dritten Gefäßposition (P3) zur gleichen Blutflusseigenschaft ($f_B$) an einer vierten Gefäßposition (P4) verwendet wird.

**15.** Verfahren gemäß einem der voranstehenden Patentansprüche 1 bis 14, **dadurch gekennzeichnet, dass** die erste Gefäßposition (P1) mindestens eine der nachfolgenden Ortsangaben oder Bedingungen erfüllt:

- die erste Gefäßposition (P1) liegt in einem venösen Gefäß,

- die erste Gefäßposition (P1) liegt in einer Armvene,
- die erste Gefäßposition (P1) liegt am Handrücken,
- die erste Gefäßposition (P1) liegt an einem zentralvenösen Katheter,
- die erste Gefäßposition (P1) liegt zwischen Handrücken und V. axillari,
- die erste Gefäßposition (P1) liegt zwischen Fuß und V. saphena magna
- die erste Gefäßposition (P1) liegt in einem zentralvenösen Gefäß.

**16.** Verfahren gemäß einem der voranstehenden Patentansprüche 1 bis 15, **dadurch gekennzeichnet, dass** die zweite Gefäßposition (P2) mindestens eine der nachfolgenden Ortsangaben oder Bedingungen erfüllt:

- die zweite Gefäßposition (P2) liegt in einem arteriellen Gefäß,
- die zweite Gefäßposition (P2) liegt in einer Beinarterie,
- die zweite Gefäßposition (P2) liegt flussabwärts zur dritten Gefäßposition (P3),
- die zweite Gefäßposition (P2) liegt flussabwärts der Bifurcation,
- die zweite Gefäßposition (P2) liegt in einer peripheren Arterie, vorzugsweise im Kniebereich,
- die zweite Gefäßposition (P2) liegt im Armbereich,
- die zweite Gefäßposition (P2) liegt im Halsbereich.

**17.** Verfahren gemäß einem der voranstehenden Patentansprüche 1 bis 16, **dadurch gekennzeichnet, dass** die dritte Gefäßposition (P3) mindestens eine der nachfolgenden Ortsangaben oder Bedingungen erfüllt:

- die dritte Gefäßposition (P3) ist die zweite Gefäßposition (P2),
- die dritte Gefäßposition (P3) liegt in der Aorta thorakalis,
- die dritte Gefäßposition (P3) liegt in der Aorta abdominalis,
- die dritte Gefäßposition (P3) liegt zwischen Aorta thorakalis und (P2)
- die dritte Gefäßposition (P3) liegt vor der Bifurcation,
- die dritte Gefäßposition (P3) liegt zwischen Bifurcation und (P2).

**18.** Verfahren gemäß einem der voranstehenden Patentansprüche 14 bis 17, **dadurch** g e **kennzeichnet**, dass die vierte Gefäßposition (P4) mindestens eine der nachfolgenden Ortsangaben oder Bedingungen erfüllt:

- die vierte Gefäßposition (P4) liegt flussaufwärts der dritten Gefäßposition (P3),
- die vierte Gefäßposition (P4) liegt flussabwärts zwischen der dritten Gefäßposition (P3) und der zweiten Gefäßposition (P2),
- die vierte Gefäßposition (P4) liegt flussabwärts der zweiten Gefäßposition (P2).

**19.** Verfahren gemäß einem der voranstehenden Patentansprüche 1 bis 18, **dadurch gekennzeichnet, dass** die Breite des Kontrastmittelbolusverlaufes (B(t)) im Bereich von 1 bis 20 Sekunden, vorzugsweise 3 bis 15 Sekunden, liegt.

**20.** Verfahren gemäß einem der voranstehenden Patentansprüche 1 bis 19, **dadurch gekennzeichnet, dass** die Korrelation der Verbreiterung ($\Delta W=W2-W1$) mit dem Flussparameter ($P_G$) einer gespeicherten Tabelle (LUT) entnommen wird.

**21.** Verfahren gemäß dem voranstehenden Patentanspruch 20, **dadurch gekennzeichnet, dass** die aus Referenzmessungen vorbestimmte Tabelle (LUT) auch mindestens einen Patientenparameter ($P_P$) aus der in Anspruch 11 genannten Liste enthält.

**22.** Verfahren gemäß einem der voranstehenden Patentansprüche 1 bis 21, **dadurch gekennzeichnet, dass** die Korrelation der Verbreiterung ($\Delta W=W2-W1$) mit dem Flussparameter ($P_G$) durch eine aus Referenzmessungen vorbestimmte Funktion ($f(P_G, P_P,)$) berechnet wird.

**23.** Verfahren gemäß dem voranstehenden Patentanspruch 22, **dadurch gekennzeichnet, dass** die aus Referenzmessungen vorbestimmte Funktion ($f(P_G, P_P)$) auch mindestens einen Patientenparameter ($P_P$) als Variable enthält.

**24.** Magnetresonanzsystem zur Erzeugung einer kontrastmittelunterstützten MR-Aufnahme oder einer MR-Gefäßdarstellung mit einem, von einem Computer gesteuerten Kontrastmittelinjektor, der Computer mit einem Speicher für Programme, die im Betrieb ausgeführt werden, ausgestattet ist, **dadurch** g e **kenn - zeichne**t , dass Programme gespeichert sind, welche das Verfahren gemäß einem der voranstehenden Verfahrensansprüche ausführen.

**25.** Computer, zumindest aufweisend einen Speicher für Programme, welche im Betrieb ausgeführt werden, dadurch gekennzeichnet, dass dort Programme gespeichert sind, welche das erfindungsgemäße Verfahren gemäß einem der voranstehenden Verfahrensansprüche ausführen.

FIG 1

# FIG 2

→ arterieller Kreislauf
*arterial circulation*

⇢ venöser Kreislauf
*venous circulation*

Lunge
*lung*

Herz
*heart*

P4

P3

innere Organe
*inner organs*

periphere Gefäße
*peripheral vessels*

P2

ROI

P1

# FIG 3

Gefäßposition P1
*vessel position*

B /
a.u.

W1

B(t)

t / a.u.

Gefäßposition P2
*vessel position*

K /
a.u.

W2 = W1 + $\Delta$W

K(t)

t / a.u.

## FIG 4

$v_G(Ad)=33$ ml/s

W1:=1s
W2=4,5s

## FIG 5

$v_G(Ad)=23$ ml/s

W1:=1s
W2=7,5s

**Europäisches Patentamt**
**European Patent Office**
**Office européen des brevets**

# EUROPÄISCHER RECHERCHENBERICHT

Nummer der Anmeldung

EP 15 16 7568

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2003/036694 A1 (LIU KECHENG [US]) 20. Februar 2003 (2003-02-20) * (par: (0001)-(0003), (0013)-(0015), (0025), Ansprüche 14-19; Fig. 1, Ansprüche 1-12; par. (0028)-(0032); ganzes Dokument * | 1-24 | INV. G01R33/28 G01R33/563 A61B5/00 |
| A | FOO T K F ET AL: "AUTOMATED DETECTION OF BOLUS AND INITIATION OF DATA ACQUISITION IN FAST, THREE-DIMENSIONAL, GADOLINIUM-ENHANCED MR ANGIOGRAPHY", RADIOLOGY, RADIOLOGICAL SOCIETY OF NORTH AMERICA, INC, US, Bd. 203, 1. Januar 1997 (1997-01-01), Seiten 275-280, XP000921005, ISSN: 0033-8419 * das ganze Dokument * | 1-24 | |
| A | US 5 590 654 A (PRINCE MARTIN R [US]) 7. Januar 1997 (1997-01-07) * das ganze Dokument * | 1-24 | RECHERCHIERTE SACHGEBIETE (IPC) |
| A | US 6 425 864 B1 (FOO THOMAS K F [US] ET AL) 30. Juli 2002 (2002-07-30) * das ganze Dokument * | 1-24 | G01R A61B |

-/--

Der vorliegende Recherchenbericht wurde für alle Patentansprüche erstellt

| Recherchenort | Abschlußdatum der Recherche | Prüfer |
|---|---|---|
| München | 3. Juli 2015 | Schindler-Bauer, P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

 
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung

EP 15 16 7568

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | L H Cheong ET AL: "An automatic approach for estimating bolus arrival time in dynamic contrast MRI using piecewise continuous regression models Home Search Collections Journals About Contact us My IOPscience An automatic approach for estimating bolus arrival time in dynamic contrast MRI using piecewise continuous regr", PHYSICS IN MEDICINE AND BIOLOGY Phys. Med. Biol, 1. Januar 2003 (2003-01-01), Seiten 83-88, XP55199846, Gefunden im Internet: URL:http://iopscience.iop.org/0031-9155/48/5/403/pdf/0031-9155_48_5_403.pdf [gefunden am 2015-07-02] * das ganze Dokument * ----- | 1-24 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| München | 3. Juli 2015 | Schindler-Bauer, P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

&  : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 15 16 7568

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

03-07-2015

| Im Recherchenbericht angeführtes Patentdokument | | Datum der Veröffentlichung | Mitglied(er) der Patentfamilie | | | Datum der Veröffentlichung |
|---|---|---|---|---|---|---|
| US 2003036694 | A1 | 20-02-2003 | EP | 1420696 | A1 | 26-05-2004 |
| | | | JP | 2004538080 | A | 24-12-2004 |
| | | | US | 2003036694 | A1 | 20-02-2003 |
| | | | WO | 03015633 | A1 | 27-02-2003 |
| US 5590654 | A | 07-01-1997 | AU | 1429297 | A | 28-07-1997 |
| | | | DE | 69607327 | D1 | 27-04-2000 |
| | | | DE | 69607327 | T2 | 09-11-2000 |
| | | | DE | 69630245 | D1 | 06-11-2003 |
| | | | DE | 69630245 | T2 | 05-08-2004 |
| | | | EP | 0812151 | A1 | 17-12-1997 |
| | | | EP | 0967491 | A2 | 29-12-1999 |
| | | | EP | 1361456 | A2 | 12-11-2003 |
| | | | HK | 1061432 | A1 | 14-01-2011 |
| | | | US | 5590654 | A | 07-01-1997 |
| | | | US | 5792056 | A | 11-08-1998 |
| | | | US | 6240311 | B1 | 29-05-2001 |
| | | | US | 2001034483 | A1 | 25-10-2001 |
| | | | US | 2003047083 | A1 | 13-03-2003 |
| | | | US | 2003163036 | A1 | 28-08-2003 |
| | | | US | 2004210130 | A1 | 21-10-2004 |
| | | | US | 2011054301 | A1 | 03-03-2011 |
| | | | WO | 9724064 | A1 | 10-07-1997 |
| US 6425864 | B1 | 30-07-2002 | EP | 1045255 | A1 | 18-10-2000 |
| | | | JP | 4693205 | B2 | 01-06-2011 |
| | | | JP | 2000308627 | A | 07-11-2000 |
| | | | US | 6425864 | B1 | 30-07-2002 |
| | | | US | 2002091316 | A1 | 11-07-2002 |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

**In der Beschreibung aufgeführte Patentdokumente**

- DE 102013204994 A1